# EUROPEAN PATENT APPLICATION

(11) **EP 4 603 297 A1**
(43) Date of publication of application: **20.08.2025**
(21) Application number: 23877242.0
(22) Date of filing: 06.10.2023
(51) Int. Cl.: B43K 8/03, A61K 8/02, C09K 3/00

(54) **LIQUID STORAGE TUBE FILLED WITH LIQUID TO BE SUPPLIED**

(30) Priority: 12.10.2022 JP 2022164126
(71) Applicant: MITSUBISHI PENCIL COMPANY, LIMITED, Tokyo 140-8537 (JP)
(72) Inventor: TAKADA, Koji, Tokyo 140-8537 (JP); ICHIKAWA, Shuji, Tokyo 140-8537 (JP)
(74) Representative: Vossius & Partner Patentanwälte Rechtsanwälte mbB
(86) International application number: PCT/JP2023/036526
(87) International publication number: WO 2024/080238

(57) **Abstract**

As illustrated in fig. 1, a liquid storage tube 100 filled with a liquid to be supplied according to the present invention comprises a liquid supply part 10 and a liquid storage part 20. In the liquid storage part 20, a to-be-supplied liquid 30 in contact with the liquid supply part 10, and a multi-layer following body 40 constituted by a plurality of layers are stored in this order from the liquid supply part 10 side. The multi-layer following body 40 includes at least a first following body 42 and a second following body 44, the first following body 42 being present further to the liquid supply part 10 side than the second following body 44. A phase angle of the first following body 42 exceeds 45° and a phase angle of the second following body 44 is 45° or less.

## Description

### FIELD

The present invention relates to a liquid storage tube filled with liquid to be supplied.

### BACKGROUND

Conventionally, following bodies (followers) have been used in storage tubes for feeding and storing liquids to be supplied such as inks for writing materials, liquid cosmetics, or liquid fuels, in order to inhibit such liquids to be supplied from being distilled, adhering to inner walls of such storage tubes, and/or the like. These following bodies move toward supply parts of the liquids to be supplied according to decrease of the liquids to be supplied due to consumption.

PTL 1 discloses an ink following body in which two gel-like materials different in viscous properties due to the difference between the viscosities of base oils are used in combination in a water-based ballpoint pen having an ink storage tube in the form of a cylinder having an inner diameter of 3 mm or more or in a form similar thereto.

PTL 2 discloses a writing material in which an oil-based ink for the writing material is stored in an ink storage tube, a pen tip is attached to an ink outflow section at one end of the ink storage tube, and an ink following body is stored in the ink storage tube so as to be in contact with the oil-based ink. The ink following body consists of at least a first layer in contact with the oil-based ink and a second layer in contact with the first layer, and the first layer is a water-based gel-like substance and the second layer is a refractory or non-volatile organic liquid.

PTL 3 discloses an ink following body composition comprising at least a mixture of one, or two or more selected from a hydrocarbon-based organic solvent having a specific gravity in a range of 0.8 to 0.9, a gelling agent, and a particle which is not substantially dissolved or swollen in the hydrocarbon-based organic solvent and which has a smaller specific gravity than that of the hydrocarbon-based organic solvent.

PTL 4 discloses a following body composition for cosmetic application tools, the composition comprising at least a base oil, and 3.5 to 8% by mass of an elastomer having a styrene structure.

PTL 5 discloses a cosmetic application tool provided with a cylindrical filling room, an application section fixed to a tip of the cylindrical filling room, a liquid cosmetic directly filled in the cylindrical filling room communicated with the application section directly or via an interposing member, and a following body of a liquid which is in contact with the liquid cosmetic, which moves according to decrease of the liquid cosmetic due to consumption, and which is different in hue from the liquid cosmetic, in which the volume of the following body is 0.5 or more under the assumption that the volume of the liquid cosmetic is 1.

PTL 6 discloses a fuel container comprising a container body having an outflow port, a fuel provided in the container body, and a highly viscous liquid provided so as to be in contact with the fuel at an opposite location to the outflow port in the container body.

### CITATION LIST

### PATENT LITERATURE

PTL 1: Japanese Unexamined Patent Publication No. H6-328890
PTL 2: Japanese Unexamined Patent Publication No. 2001-158869
PTL 3: Japanese Unexamined Patent Publication No. 2005-239918
PTL 4: Japanese Unexamined Patent Publication No. 2017-165670
PTL 5: Japanese Unexamined Patent Publication No. 2016-198395
PTL 6: Japanese Unexamined Patent Publication No. 2004-281340

### SUMMARY

### TECHNICAL PROBLEM

The present invention provides a novel liquid storage tube filled with a liquid to be supplied which has impact resistance and which can inhibit a following body from adhering to the liquid storage tube according to decrease of the liquid to be supplied due to consumption and from being leaked when placed inversely.

### SOLUTION TO PROBLEM

The present inventors have made intensive studies, and thus have found that the above problems can be solved by the following solutions and have completed the present invention. In other words, the present invention is as follows:
<Aspect 1> A liquid storage tube filled with a liquid to be supplied comprising a liquid supply part and a liquid storage part, wherein
   in the liquid storage part, a liquid to be supplied in contact with the liquid supply part and a multi-layer following body which consists of a plurality of layers are stored in this order from the liquid supply part side,
   the multi-layer following body comprises at least a first following body and a second following body, and the first following body is located closer to the liquid supply part than the second following body,
   a phase angle of the first following body, measured according to JIS K 7244, is more than 45°, and
   a phase angle of the second following body, measured according to JIS K 7244, is 45° or less.
<Aspect 2> The liquid storage tube filled with a liquid to be supplied according to Aspect 1, wherein the first following body is in contact with the liquid to be supplied.
<Aspect 3> The liquid storage tube filled with a liquid to be supplied according to Aspect 1 or 2, wherein the second following body is in contact with the first following body.
<Aspect 4> The liquid storage tube filled with a liquid to be supplied according to any one of Aspects 1 to 3, wherein the multi-layer following body consists of the first following body and the second following body.
<Aspect 5> The liquid storage tube filled with a liquid to be supplied according to any one of Aspects 1 to 4, wherein an interface between the following bodies constituting the multi-layer following body is viewable.
<Aspect 6> The liquid storage tube filled with a liquid to be supplied according to any one of Aspects 1 to 5, wherein a difference between the phase angle of the first following body and the phase angle of the second following body is 10° or more.
<Aspect 7> The liquid storage tube filled with a liquid to be supplied according to any one of Aspects 1 to 6, wherein the first following body contains a first base oil and a first thickener.
<Aspect 8> The liquid storage tube filled with a liquid to be supplied according to Aspect 7, wherein the first base oil is at least one selected from the group consisting of polyolefin, liquid paraffin, and mineral oil.
<Aspect 9> The liquid storage tube filled with a liquid to be supplied according to Aspect 7 or 8, wherein the first thickener is at least one selected from silica and an inorganic salt particle.
<Aspect 10> The liquid storage tube filled with a liquid to be supplied according to any one of Aspects 1 to 9, wherein the second following body contains a second base oil and a second thickener.
<Aspect 11> The liquid storage tube filled with a liquid to be supplied according to Aspect 10, wherein the second base oil is at least one selected from the group consisting of polyolefin, liquid paraffin, and mineral oil.
<Aspect 12> The liquid storage tube filled with a liquid to be supplied according to Aspect 10 or 11, wherein the second thickener is an elastomer.

### ADVANTAGEOUS EFFECTS OF INVENTION

According to the present invention, it is possible to provide a novel liquid storage tube filled with a liquid to be supplied which has impact resistance and which can inhibit a following body from adhering to the liquid storage tube according to decrease of the liquid to be supplied due to consumption and from being leaked when placed inversely.

### BRIEF DESCRIPTION OF DRAWING

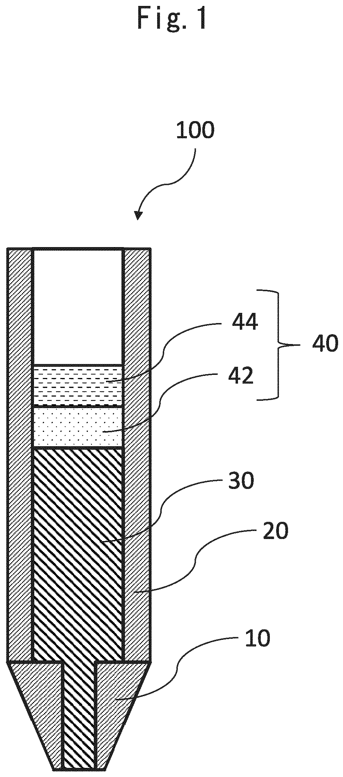

FIG. 1 [ 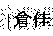 1]is a sectional side view of the liquid storage tube filled with a liquid to be supplied of the present invention.

### DESCRIPTION OF EMBODIMENTS

### << Liquid storage tube filled with a liquid to be supplied >>

As illustrated in FIG. 1, a liquid storage tube filled with a liquid to be supplied 100 of the present invention comprises a liquid supply part 10 and a liquid storage part 20, wherein
in the liquid storage part 20, a liquid to be supplied 30 in contact with the liquid supply part 10 and a multi-layer following body 40 which consists of a plurality of layers are stored in this order from the liquid supply part 10 side,
the multi-layer following body 40 comprises at least a first following body 42 and a second following body 44, and the first following body 42 is located closer to the liquid supply part 10 than the second following body 44,
the phase angle of the first following body 42, measured according to JIS K 7244-10, is more than 45°, and
the phase angle of the second following body 44, measured according to JIS K 7244-10, is 45° or less.

In the present invention, the "phase angle" is measured according to JIS K 7244-10. Specifically, the phase angle can be measured by a strain control system under conditions of a temperature of 25°C, a shear strain of 1%, and a frequency of 1 Hz.

While a following body in which the phase angle is more than 45° generally has the property more similar to that of a viscous body, a following body in which the phase angle is 45° or less generally has the property more similar to that of an elastic body.

A following body more similar to a viscous body has the advantage of being excellent in impact resistance. More specifically, when impact is applied to a liquid storage tube filled with a liquid to be supplied having this following body, this following body absorbs the impact and as a result, can inhibit a liquid to be supplied from penetrating through this following body and thus being leaked outward. On the other hand, this following body adheres to a wall portion of the liquid storage tube when moved due to consumption of the liquid to be supplied, and as a result, this following body cannot protect the liquid to be supplied in some cases. This following body, when placed inversely, specifically, left to still stand in the state of being located below the liquid to be supplied, may be changed in shape by the influence of gravity and leaked via the wall portion of the liquid storage tube.

A following body more similar to an elastic body, although suppressed in the problem of adhesion to a wall portion of a liquid storage tube, is not sufficient in impact resistance, and may cause leakage of a liquid to be supplied due to penetration of the liquid to be supplied through this following body with an impact force in application of impact to a liquid storage tube filled with a liquid to be supplied having this following body.

On the contrary, the present inventors have found that following bodies having such properties can be combined to provide a following body which has impact resistance and which can be inhibited from adhering to a liquid storage tube due to consumption of a liquid to be supplied and from being leaked when placed inversely.

The difference between the phase angle of the first following body and the phase angle of the second following body is preferably 10° or more from the viewpoint that the above effects are favorably obtained. The difference may be 15° or more, 20° or more, 25° or more, or 30° or more.

Hereinafter, each of the constituent components in the present invention is described.

### <Liquid supply part and liquid storage part >

The liquid supply part is a member for feeding the liquid to be supplied to the outside of the liquid storage tube. The liquid storage part is a member for storing the liquid to be supplied. The liquid supply part and the liquid storage part may be connected.

The material constituting the liquid storage part is not particularly limited, and a resin such as polypropylene, paper, a metal, and/or the like can be used. The material here used may be a biomass plastic in which a biological source such as corn or sugarcane is used, or a biodegradable plastic in which a soil-biodegradable resin or a marine-biodegradable resin is used.

For example, in a case where the liquid storage tube filled with a liquid to be supplied of the present invention is a refill for writing materials, the liquid supply part and the liquid storage part in the present invention may be a writing section and an ink storage part respectively.

### <Liquid to be supplied>

The liquid to be supplied may be any liquid to be supplied, and may be, for example, an ink, a liquid fuel, a liquid cosmetic product, or the like.

The ink may be a water-based ink, may be an oil-based ink, or may be a gel ink.

Herein, in general, the water-based ink refers to an ink containing at least water and a coloring material, the oil-based ink refers to an ink containing at least an organic solvent and a coloring material, and the gel ink refers to such a water-based ink further containing a viscosity modifier.

### <Multi-layer following body>

The multi-layer following body is a following body which consists of a plurality of layers. The multi-layer following body comprises at least a first following body and a second following body.

The first following body in the present invention is preferably in contact with the liquid to be supplied from the viewpoint that favorable impact resistance is obtained.

The second following body in the present invention is preferably in contact with the first following body from the viewpoint that the following bodies are inhibited from adhering to the liquid storage tube due to consumption of the liquid to be supplied and from being leaked when placed inversely.

In particular, the multi-layer following body may consist of the first following body and the second following body.

The interface between the following bodies constituting the multi-layer following body may be clearly unviewable like gradation, or may be clearly viewable. At least one of the following bodies may be colored by a color material not dissolved or eluted in the liquid to be supplied. In a case where a plurality of such following bodies is colored, such following bodies preferably have respective different colors from the viewpoint of design and viewability. A clearly viewable interface can be obtained by, for example, allowing the difference between the phase angle of the first following body and the phase angle of the second following body to be large, for example, to be 10° or more.

### (First following body)

The first following body is a following body located closer to the liquid supply part, than the second following body.

The phase angle of the first following body, as measured according to JIS K 7244, is more than 45°. The phase angle may be 47° or more, 50° or more, 52° or more, 55° or more, or 60° or more.

The first following body may contain a first base oil and a first thickener.

The first base oil here used can be, for example, a mineral oil, a synthetic hydrocarbon oil, an ester oil, and a polyglycol oil. These may be used singly or as a mixture thereof.

The mineral oil here used can be, for example, a spindle oil and Vaseline.

The synthetic hydrocarbon oil here used can be, for example, a polyolefin such as polybutene, an olefin oligomer, an aromatic hydrocarbon, and liquid paraffin.

The ester oil and the polyglycol oil here used can be those commonly used for following bodies.

The first thickener is preferably at least one thickener selected from silica and an inorganic salt particle from the viewpoint that the above phase angle is obtained. These may be used singly or as a mixture thereof.

The inorganic salt particle here used can be, for example, clay minerals such as kaolinite, smectite, sericite, illite, glauconite, chlorite, talc, and zeolite, and water-insoluble metal soaps such as lithium stearate, aluminum stearate, and sodium stearate.

The content of the first thickener based on the mass of the first following body may be 1% by mass or more, 2% by mass or more, 3% by mass or more, or 4% by mass or more, and may be 10% by mass or less, 9% by mass or less, 8% by mass or less, 7% by mass or less, or 6% by mass or less.

The first following body can be obtained by kneading each material constituting the following body under warming, with a kneading tool such as a planetary mixer or a roll mill, and then cooling the kneaded product.

Here, the phase angle of this following body can be increased by changing the composition blending ratio, production conditions, and the like of this following body, and specifically can be increased by increasing the cooling rate and/or increasing the stirring strength.

### <Second following body>

The second following body is a layer located opposite to the liquid supply part, as compared with the first following body.

The phase angle of the second following body, as measured according to JIS K 7244, is 45° or less. The phase angle may be 42° or less, 40° or less, 37° or less, 35° or less, 33° or less, or 30° or less.

The second following body may contain a second base oil and a second thickener.

The second base oil here used can be one exemplified with respect to the first base oil.

The second thickener here used is preferably an elastomer from the viewpoint that the above phase angle is obtained.

The elastomer here used can be, for example, an olefin-based thermoplastic elastomer such as an ethylene-ethylene/butylene-ethylene block copolymer, a styrene-based thermoplastic elastomer such as a styrene-ethylene/butylene-styrene block copolymer, a vinyl chloride-based thermoplastic elastomer, a polyamide-based thermoplastic elastomer, a polyester-based thermoplastic elastomer, and a polyurethane-based thermoplastic elastomer. These may be used singly or as a mixture thereof.

The content of the second thickener based on the mass of the second following body may be 1% by mass or more, 2% by mass or more, 3% by mass or more, or 4% by mass or more, and may be 10% by mass or less, 9% by mass or less, 8% by mass or less, 7% by mass or less, or 6% by mass or less. In particular, in a case where the second thickener is the elastomer, the content of the elastomer is preferably 2% by mass or more from the viewpoint that the above phase angle is obtained.

The second following body can be produced by a method exemplified with respect to the first following body.

Here, the phase angle of this following body can be increased by changing the composition blending ratio, production conditions, and the like of this following body, and the phase angle can be decreased in an opposite manner to the manner for increasing the phase angle, specifically, by decreasing the cooling rate and/or decreasing the stirring strength.

### <Other constituent>

The liquid storage tube filled with a liquid to be supplied of the present invention optionally has any other constituent. Such any other constituent here used can be, for example, a solid embedded in the following body(s), like a follower rod. The follower rod may be present so as to be embedded in the multi-layer following body. More specifically, a plurality of such follower rods may be present so as to be embedded in the respective following bodies constituting the multi-layer following body, or one such follower rod may be embedded so as to run through the multi-layer following body.

### EXAMPLES

The present invention is specifically described with reference to Examples and Comparative Examples, but the present invention is not limited thereto.

### <<Production of liquid storage tube filled with a liquid to be supplied >>

The first base oil and the first thickener in the types and amounts shown in Table 1 were kneaded with a planetary mixer and then kneaded with a roll mill, thereby producing the first following body.

The second base oil and the second thickener in the types and amounts shown in Table 1 were kneaded with a planetary mixer and then kneaded with a roll mill, thereby producing the second following body.

The details of base oils shown in Table 1 are as follows:
Polybutene A: Polybutene 015N, NOF CORPORATION
Polybutene B: Polybutene 30N, NOF CORPORATION
Mineral oil A: DIANA PROCESS OIL PW-90, Idemitsu Kosan Co., Ltd.
Mineral oil B: DIANA PROCESS OIL PW-90, Idemitsu Kosan Co., Ltd.
Aromatic substance: BARREL PROCESS OIL B-05, Matsumura Oil Co., Ltd.
Paraffin: BARREL PROCESS OIL P-2200, Matsumura Oil Co., Ltd.

The details of thickeners shown in Table 1 are as follows:
Elastomer A: DYNARON 6200P, JSR Corporation
Elastomer B: DYNARON 8300P, JSR Corporation
Inorganic salt: lithium stearate
Silica A: AEROSIL R202, Evonik Industries
Silica B: AEROSIL NY50L, Evonik Industries

An ink-containing refill was produced by enclosing an ink shown in the column "Liquid to be supplied (ink)" in Table 1, as the liquid to be supplied, into a liquid storage tube shown in Table 1, and then enclosing the first following body and the second following body in this order.

The phase angle of each of the resulting following bodies was measured by a strain control system under conditions of a temperature of 25°C, a shear strain of 1%, and a frequency of 1 Hz, according to JIS K 7244-10.

The water-based ink indicates an ink in which water is used as a main solvent and which contains at least a colorant, and is mentioned as "Water-based" in the column "Liquid to be supplied (ink)" in Table 1.

"Gel" mentioned in the column "Liquid to be supplied (ink)" in Table 1 indicates an ink in which water is used as a main solvent and which contains at least a colorant/viscosity modifier.

"Oil-based" mentioned in the column "Liquid to be supplied (ink)" in Table 1 indicates an ink in which an organic solvent is used as a main solvent and which contains at least a colorant.

"PP" mentioned in the column "Storage tube" in Table 1 indicates a refill (inner diameter 5 mm) comprising an ink storage part made of polypropylene.

"Paper" mentioned in the column "Storage tube" in Table 1 indicates a refill (inner diameter 5 mm) comprising an ink storage part having a paper layer as a base layer.

"Metal" in the column "Storage tube" in Table 1 indicates a refill (inner diameter 5 mm) comprising a metallic ink storage part.

### <<Evaluation>>

### <Impact resistance>

The state of the following bodies was confirmed by fixing a 10-g weight to a rear end of the resulting ink-containing refill and dropping the refill in a state where a pen tip was positioned upward, from a height of 1 m, and observing the refill from the outside, or disassembling the refill with a cutter in the longitudinal direction and observing the resultant, to visually confirm the states of the ink and the following bodies. The evaluation criteria are as follows.
A: no incorporation of the ink to the following bodies was confirmed.
B: the shape of the interface between the ink and the following bodies was broken.
C: the ink penetrated through the interior of the following bodies.

### <Adhering of following body to wall surface >

Whether or not the following bodies adhered to a wall surface was confirmed by performing writing with the ink-containing refill produced, until the amount of filling with the ink reached 5 to 10% of the initial amount, and observing the refill from the outside, or disassembling the refill with a cutter in the longitudinal direction and observing the resultant, to visually confirm the state of the ink and the following bodies. The evaluation criteria are as follows.
A: no adhering of the following bodies to the wall surface was confirmed.
B: although adhering of the following bodies to the wall surface was found, the length of filling of the following bodies after writing was 70% or more of the initial length of filling.
C: adhering of the following bodies to the wall surface was found, and the length of filling of the following bodies of the writing material was less than 70% of the initial length of filling.

### <Leakage resistance>

The sagging of the following bodies to a wall surface was confirmed by disposing the ink-containing refill produced, so that the following bodies were located under the ink, leaving the resultant to still stand for 90 days, and observing the refill from the outside, or disassembling the refill with a cutter in the longitudinal direction and observing the resultant, to visually confirm the state of the ink and the following bodies. The evaluation criteria are as follows.
A: no change in state as compared with the state before the standing was found.
B: the sagging of the following bodies could be clearly confirmed.
C: the sagging of the following bodies reached the vicinity of the inlet of the refill.

Configurations and evaluation results in Examples and Comparative Examples are shown in Table 1.

### [Table 1]

**Table 1**

| | | | | Example 1 | Example 2 | Example 3 | Example 4 | Example 5 | Example 6 | Comparative Example 1 | Comparative Example 2 | Comparative Example 3 |
|---|---|---|---|---|---|---|---|---|---|---|---|---|
| | | First base oil | Type | Polybutene B | Aromatic substance | Aromatic substance | Polybutene A | Polybutene B | Paraffin | Polybutene B | Aromatic substance | Polybutene B |
| | | | Content (% by mass) | 15.0 | 38.7 | 95.5 | 85.3 | 24.6 | 96.2 | 21.8 | 96.8 | 64.0 |
| | | | Type | Mineral oil B | Paraffin | | Mineral oil A | Mineral oil A | | Mineral oil B | | Mineral oil A |
| | | | Content (% by mass) | 81.5 | 58.0 | | 11.8 | 71.1 | | 74.0 | | 33.7 |
| | First following body | First thickener | Type | Inorganic salt | Inorganic salt | Inorganic salt | Silica B | Inorganic salt | Silica B | Silica A | Inorganic salt | Elastomer B |
| | | | Content (% by mass) | 2.4 | 1.9 | 1.0 | 2.9 | 1.5 | 3.8 | 4.2 | 3.2 | 2.3 |
| | | | Type | Silica A | Silica B | Silica A | | Silica A | | | | |
| | | | Content (% by mass) | 1.1 | 1.4 | 2.8 | | 2.8 | | | | |
| | | Presence of roll mill | | Presence | Presence | Presence | Presence | Presence | Absence | Absence | Presence | Absence |
| | | Phase angle (°) | | 52 | 63 | 55 | 65 | 54 | 70 | 64 | 72 | 30 |
| Configuration | Second following body | Second base oil | Type | Aromatic substance | Polybutene A | Polybutene B | Paraffin | Aromatic substance | Polybutene A | - | Polybutene B | Aromatic substance |
| | | | Content (% by mass) | 57.6 | 55.8 | 23.0 | 95.9 | 96.3 | 44.2 | | 22.0 | 96.4 |
| | | | Type | Paraffin | Mineral oil B | Mineral oil B | | | Mineral oil B | | Mineral oil A | |
| | | | Content (% by mass) | 40.0 | 40.3 | 72.4 | | | 52.0 | | 76.2 | |
| | | Second thickener | Type | Elastomer A | Elastomer B | Elastomer B | Elastomer A | Elastomer B | Elastomer A | | Silica A | Elastomer A |
| | | | Content (% by mass) | 2.4 | 3.9 | 4.6 | 4.1 | 3.7 | 3.8 | | 1.8 | 3.6 |
| | | Presence of roll mill | | Absence | Presence | Absence | Presence | Presence | Presence | | Absence | Presence |
| | | phase angle (°) | | 40 | 22 | 33 | 35 | 29 | 31 | | 61 | 33 |
| | (Phase angle of first following body) - (Phase angle of following body) | | | 12 | 41 | 22 | 30 | 25 | 39 | - | 11 | -3 |
| | Liquid to be supplied (ink) | | | Water-based | Water-based | Gel | Gel | Oil-based | Oil-based | Water-based | Gel | Oil-based |
| | Liquid storage tube | | | PP | Metal | PP | Paper | PP | Paper | PP | PP | Paper |
| Evaluative results | Impact resistance | | | A | A | A | A | A | A | A | A | C |
| | Adhering of follower to wall surface | | | A | A | A | A | A | A | C | C | A |
| | Leakage resistance | | | A | A | A | A | A | A | B | C | A |

It would be understood from Table 1 that the ink-containing refill of Examples, in which the phase angle of the first following body was more than 45° and the phase angle of the second following body was 45° or less, had impact resistance and could inhibit the following bodies from adhering to the wall surface due to consumption of the liquid to be supplied and from being leaked when placed inversely.

### REFERENCE SIGNS LIST

100 Liquid storage tube filled with a liquid to be supplied
10 Liquid supply part
20 Liquid storage part
30 Liquid to be supplied
40 Multi-layer following body
42 First following body
44 Second following body

## Claims

1. A liquid storage tube filled with a liquid to be supplied comprising a liquid supply part and a liquid storage part, wherein
in the liquid storage part, a liquid to be supplied in contact with the liquid supply part and a multi-layer following body which consists of a plurality of layers are stored in this order from the liquid supply part side,
the multi-layer following body comprises at least a first following body and a second following body, and the first following body is located closer to the liquid supply part than the second following body,
a phase angle of the first following body, measured according to JIS K 7244, is more than 45°, and
a phase angle of the second following body, measured according to JIS K 7244, is 45° or less.

2. The liquid storage tube filled with a liquid to be supplied according to Claim 1, wherein the first following body is in contact with the liquid to be supplied.

3. The liquid storage tube filled with a liquid to be supplied according to Claim 1 or 2, wherein the second following body is in contact with the first following body.

4. The liquid storage tube filled with a liquid to be supplied according to any one of Claims 1 to 3, wherein the multi-layer following body consists of the first following body and the second following body.

5. The liquid storage tube filled with a liquid to be supplied according to any one of Claims 1 to 4, wherein an interface between the following bodies constituting the multi-layer following body is viewable.

6. The liquid storage tube filled with a liquid to be supplied according to any one of Claims 1 to 5, wherein a difference between the phase angle of the first following body and the phase angle of the second following body is 10° or more.

7. The liquid storage tube filled with a liquid to be supplied according to any one of Claims 1 to 6, wherein the first following body contains a first base oil and a first thickener.

8. The liquid storage tube filled with a liquid to be supplied according to Claim 7, wherein the first base oil is at least one selected from the group consisting of polyolefin, liquid paraffin, and mineral oil.

9. The liquid storage tube filled with a liquid to be supplied according to Claim 7 or 8, wherein the first thickener is at least one selected from silica and an inorganic salt particle.

10. The liquid storage tube filled with a liquid to be supplied according to any one of Claims 1 to 9, wherein the second following body contains a second base oil and a second thickener.

11. The liquid storage tube filled with a liquid to be supplied according to Claim 10, wherein the second base oil is at least one selected from the group consisting of polyolefin, liquid paraffin, and mineral oil.

12. The liquid storage tube filled with a liquid to be supplied according to Claim 10 or 11, wherein the second thickener is an elastomer.
